# EUROPEAN PATENT APPLICATION

(11) **EP 4 674 353 A1**
(43) Date of publication of application: **07.01.2026**
(21) Application number: 25186792.5
(22) Date of filing: 01.07.2025
(51) Int. Cl.: A61B 6/03, A61B 6/42, A61B 6/00

(54) **INFORMATION PROCESSING APPARATUS, INFORMATION PROCESSING METHOD, AND PROGRAM**

(30) Priority: 02.07.2024 JP 2024107036
(71) Applicant: FUJIFILM Corporation, Tokyo 106-8620 (JP)
(72) Inventor: ITAI, Yoshinori, Tokyo, 1068620 (JP)
(74) Representative: Dehns Germany Partnerschaft mbB

(57) **Abstract**

Provided are an information processing apparatus, an information processing method, and a program that can suppress the generation of unnecessary images in a CT apparatus and provide images useful for diagnosis in a time-efficient manner. An information processing apparatus (10) includes a processor (102), in which the processor (102) is configured to: acquire a first image captured by a CT apparatus (30) ; execute an analysis process of the first image using a learning device; cause the CT apparatus (30) to execute a generation process of a second image corresponding to a result of the analysis process; and display at least one of the first image, the result of the analysis process, or the second image on a display device (124).

## Description

### BACKGROUND OF THE INVENTION

### 1. Field of the Invention

The present disclosure relates to an information processing apparatus, an information processing method, and a program, and in particular to an image processing technology for processing images captured by a computed tomography (CT) apparatus, and a technology for improving an image diagnosis workflow.

### 2. Description of the Related Art

JP2021-10727A describes an X-ray CT system that uses two or more types of X-ray energy to discriminate between materials such as kidney stones, fat, or bones within the body of a subject. The X-ray CT system described in JP2021-10727A comprises a scanning unit that executes a first scan to collect a first subject data set corresponding to a first X-ray energy by irradiating a first area of a subject with X-rays, and executes a second scan after the first scan to collect a second subject data set corresponding to a second X-ray energy and a third subject data set corresponding to a third X-ray energy different from the second X-ray energy by irradiating a second area included in the first area with X-rays; and a processing unit that performs material discrimination using a plurality of reference materials based on a fourth subject data set obtained based on the first subject data set and one of the second and third subject data sets, and the other of the second and third subject data sets. The "X-ray CT system" corresponds to the "CT apparatus" in the present specification.

JP6747787B describes a photon counting X-ray CT apparatus capable of performing material discrimination with high accuracy. The photon counting X-ray CT apparatus described in JP6747787B comprises a data collection unit that collects a first data group, which is count data for each of a plurality of first energy bands, by allocating energy measured by a signal output by a photon counting detector in response to the incidence of X-ray photons to one of the plurality of first energy bands; a determination unit that determines a plurality of second energy bands into which the plurality of first energy bands are grouped according to a discrimination target material, which is a material to be discriminated in an imaging part; an allocation unit that generates a second data group by allocating the first data group to one of the plurality of second energy bands; and a generation unit that generates an image related to the distribution of the discrimination target material using the second data group, in which widths of the plurality of second energy bands are different from each other, and the determination unit selects the widths of the plurality of second energy bands such that the integral value of the X-ray absorption rate in the imaging part in each of the plurality of second energy bands is the same. The "photon counting X-ray CT apparatus" corresponds to the "photon counting CT apparatus" in the present specification. Hereinafter, the photon counting CT apparatus will be referred to as a "PCCT apparatus".

### SUMMARY OF THE INVENTION

Images captured by a CT apparatus in a hospital or other medical institution are sent to a medical image management system referred to as a picture archiving and communication system (PACS) via a communication network such as an in-hospital network, and the images are stored, shared, viewed, and the like. In a CT apparatus that can generate images of other image types, such as material discrimination images, in addition to normal CT images, it is conceivable to automatically generate images of a plurality of image types that can be generated without selecting the images of the plurality of image types, and to store all of these images of the plurality of image types in a PACS.

While such a configuration has the advantage of being able to provide images of a plurality of image types required for diagnosis in a short period of time, the amount of image data to be stored in the PACS is enormous, which puts a strain on the storage capacity of the PACS. Furthermore, even in a case in which images of a plurality of image types are automatically generated, it is not guaranteed that all of these images will actually be used for analysis or diagnosis, and the like, and as a result, unnecessary images that are not used will be generated, transmitted, and stored. In a case in which unnecessary images that are not used for analysis or the like are transmitted to the PACS via the network, the increased traffic puts a strain on the network.

To address the issues associated with automatically generating images of a plurality of image types in a random manner, it is conceivable that the CT apparatus receives image generation instructions from a user, generates images of different image types on demand, and transmits the requested images to the PACS. However, in such an on-demand system, it takes time from when an instruction is received until an image subjected to the reconstruction process is obtained, making efficient interpretation difficult.

The present disclosure has been made in consideration of the above circumstances, and an object of the present disclosure is to provide an information processing apparatus, an information processing method, and a program that can suppress the generation of unnecessary images in a CT apparatus and provide images useful for diagnosis in a time-efficient manner.

According to a first aspect of the present disclosure, there is provided an information processing apparatus comprising a processor, in which the processor may be configured to: acquire a first image captured by a CT apparatus; execute an analysis process of the first image using a learning device; cause the CT apparatus to execute a generation process of a second image corresponding to a result of the analysis process; and display at least one of the first image, the result of the analysis process, or the second image on a display device.

According to the first aspect, based on the result of the analysis process of the first image using the learning device, a generation process of a second image corresponding to the result of the analysis process is executed. **In** this way, by using the result of the analysis process using the learning device to control the generation process of the second image in the CT apparatus, it is possible to suppress the generation of unnecessary images. Furthermore, according to the first aspect, since the result of the analysis process of the first image using the learning device can be used to cause the CT apparatus to execute the generation process of the second image, it is possible to display the second image that the user wishes to observe on the display device in a short period of time. Accordingly, it is possible to perform interpretation with high time efficiency.

According to a second aspect, in the information processing apparatus according to the first aspect, the first image may be a CT image, and the second image may be a material discrimination image.

According to a third aspect, in the information processing apparatus according to the first aspect, the second image may be an image generated by an image reconstruction process under a reconstruction condition different from a reconstruction condition of the first image.

According to a fourth aspect, in the information processing apparatus according to any one of the first to third aspects, the processor may be configured to output an instruction to the CT apparatus to generate the second image based on the result of the analysis process.

According to a fifth aspect, in the information processing apparatus according to any one of the first to fourth aspects, the processor may be configured to set a generation condition of the second image according to the result of the analysis process.

According to a sixth aspect, in the information processing apparatus according to any one of the first to fifth aspects, the learning device may include a trained model that causes the processor to execute a process of detecting a lesion from an input image.

According to a seventh aspect, in the information processing apparatus according to any one of the first to sixth aspects, the learning device may include a trained model that causes the processor to execute a process of detecting at least one of a tumor, a nodule, a stone, fat, or a calcified area from an input image.

According to an eighth aspect, in the information processing apparatus according to any one of the first to seventh aspects, the analysis process may include at least one process of detection of an adrenal tumor, detection of a urinary stone, detection of a gallstone, or calculation of a liver fat amount.

According to a ninth aspect, in the information processing apparatus according to any one of the first to eighth aspects, the processor may be configured to, in a case in which an adrenal tumor is detected by the analysis process, cause the CT apparatus to execute a generation process of a fat density image as the second image.

According to a tenth aspect, in the information processing apparatus according to the ninth aspect, the processor may be configured to set a slice thickness of the fat density image generated by the generation process according to a size of the adrenal tumor detected by the analysis process.

According to an eleventh aspect, in the information processing apparatus according to the ninth or tenth aspect, the processor may be configured to set a slice position and the number of slices of the fat density image generated by the generation process according to a position of the adrenal tumor detected by the analysis process.

According to a twelfth aspect, in the information processing apparatus according to the first to eleventh aspects, the processor may be configured to: receive a display request for the second image via an input device; and display the second image on the display device based on the display request.

According to a thirteenth aspect, the information processing apparatus according to any one of the first to twelfth aspects may further comprise a storage device including a temporary storage area and a persistent storage area, in which the processor may be configured to: store the first image in the persistent storage area; and store the second image in the temporary storage area.

According to a fourteenth aspect, in the information processing apparatus according to the thirteenth aspect, the processor may be configured to store the second image displayed on the display device in the persistent storage area.

According to a fifteenth aspect, in the information processing apparatus according to the thirteenth or fourteenth aspect, the processor may be configured to store, in the persistent storage area, only an image displayed on the display device among the second images stored in the temporary storage area.

According to a sixteenth aspect, there is provided an information processing method executed by a processor, the information processing method comprising: a step of acquiring a first image captured by a CT apparatus; a step of executing an analysis process of the first image using a learning device; a step of causing the CT apparatus to execute a generation process of a second image corresponding to a result of the analysis process; and a step of displaying at least one of the first image, the result of the analysis process, or the second image on a display device.

The processor may automatically execute the processing of each step according to a program, or may receive an input of instructions from a user for some or all of the steps and execute the processing in accordance with the received instructions.

The information processing method according to the sixteenth aspect may have a configuration including the same specific aspect as the information processing apparatus according to any one of the second to fifteenth aspects.

According to a seventeenth aspect, a program that causes a computer to implement: a function of acquiring a first image captured by a CT apparatus; a function of executing an analysis process of the first image using a learning device; a function of causing the CT apparatus to execute a generation process of a second image corresponding to a result of the analysis process; and a function of displaying at least one of the first image, the result of the analysis process, or the second image on a display device.

The program according to the seventeenth aspect may have a configuration including the same specific aspect as the information processing apparatus according to any one of the second to fifteenth aspects. The present disclosure also includes a non-transitory, tangible computer-readable storage medium having the program according to the seventeenth aspect stored therein.

According to the aspects of the present disclosure, it is possible to suppress the generation of unnecessary images in a CT apparatus, and to provide images useful for diagnosis in a time-efficient manner.

### BRIEF DESCRIPTION OF THE DRAWINGS

Fig. 1 is a block diagram showing an example of a medical information system to which an information processing apparatus according to an embodiment is applied.
Fig. 2 is a block diagram showing an example of a hardware configuration of a computer.
Fig. 3 is an explanatory diagram showing an outline of operations of the information processing apparatus and a PCCT apparatus according to the embodiment.
Fig. 4 is a flowchart showing an example of operations of the information processing apparatus and the PCCT apparatus according to the embodiment.

### DESCRIPTION OF THE PREFERRED EMBODIMENTS

Hereinafter, a preferred embodiment of the present invention will be described in detail with reference to the accompanying drawings. In the present specification, the same components are denoted by the same reference numerals, and duplicate description thereof will be omitted as appropriate.

### Example of Information Processing Apparatus

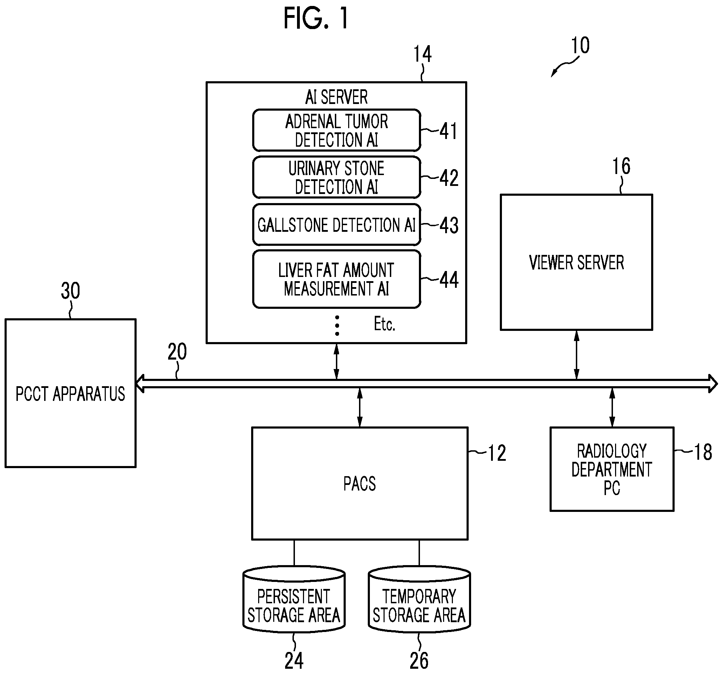

Fig. 1 is a block diagram showing an example of a medical information system including an information processing apparatus 10 according to an embodiment. The information processing apparatus 10 is a computer system including a PACS 12, an artificial intelligence (AI) server 14, and a viewer server 16, and is connected to a PCCT apparatus 30 via a communication network 20. In addition, a radiology department personal computer (PC) 18, which is an example of a terminal that can access the viewer server 16, is connected to the communication network 20. The information processing apparatus 10 may include the radiology department PC 18.

The PACS 12 is a computer system that stores and manages various types of data including medical images captured by the PCCT apparatus 30 and a modality device (not shown). The PACS 12 comprises large-capacity storage devices, including a persistent storage area 24 and a temporary storage area 26, and a database management program. The PACS 12 may include, for example, a DICOM server that operates according to digital imaging and communications in medicine (DICOM) specifications. The persistent storage area 24 is an area in which information is stored for a longer period of time than the temporary storage area 26.

The AI server 14 includes an image processing apparatus that uses AI technology to execute an image analysis process. The AI server 14 comprises one or more AI modules, and preferably a plurality of AI modules, and acquires images to be processed from the PACS 12 and executes processing by the AI modules. The AI module may be, for example, a learning device constructed using a convolutional neural network and trained to perform a task of object detection, a task of area classification (semantic segmentation), a task of numerical prediction of a measurement value, or the like.

The AI server 14 comprises a plurality of AI modules, such as an adrenal tumor detection AI 41, a urinary stone detection AI 42, a gallstone detection AI 43, and a liver fat amount measurement AI 44. The adrenal tumor detection AI 41 includes a trained model trained by machine learning to detect adrenal tumors from input CT images. The urinary stone detection AI 42 includes a trained model trained by machine learning to detect urinary stones from input CT images. The gallstone detection AI 43 includes a trained model trained by machine learning to detect gallstones from input CT images. The liver fat amount measurement AI 44 includes a trained model trained by machine learning to output a predicted value of liver fat amount from an input CT image. In addition, a trained model is essentially a program.

The PACS 12, the AI server 14, and the viewer server 16 are communicatively connected to each other via a communication network 20. The communication network 20 may be a local area network, a wide area network, or a combination of these. The communication method of the communication network 20 is not limited to wired communication, but may be wireless communication, or a combination of these.

The results of the analysis process by the AI server 14 (AI results) are sent to the viewer server 16. In addition, the AI results may be stored in the PACS 12 in association with the corresponding CT images. In the present embodiment, the AI server 14 instructs the PCCT apparatus 30 to generate a material discrimination image according to the AI results. Details thereof will be described below.

The PCCT apparatus 30 is a CT apparatus capable of reconstructing an image that has been subjected to material discrimination by counting photons of X-rays (X-ray photons) that have been transmitted through a subject using a photon counting detector. The term "material discrimination" includes the concept of discriminating the type, amount, density, and the like of a material. Since the configuration of the PCCT apparatus 30 is well known, a detailed description thereof will be omitted here. As the PCCT apparatus 30, for example, a PCCT apparatus described in JP2022-106241A or the like may be applied. The PCCT apparatus 30 can obtain the X-ray intensity for each energy bin by discriminating the counted individual X-ray photons according to their energy values. Using this, the PCCT apparatus 30 can extract and image only X-rays in a specific energy range.

The PCCT apparatus 30 includes a scanner gantry, a bed, and a console (control console). The console is configured using a computer. The computer that constitutes the console is implemented with the function of a control device that controls the imaging operation of the PCCT apparatus 30 and the function of an image processing apparatus that processes the transmitted X-ray data obtained from the scanner gantry and performs arithmetic operations for image reconstruction. The raw data acquired by the scanner gantry and the image generated by the reconstruction (the reconstructed image) are stored in a storage device built into the console. The image generated by the reconstruction is displayed on the display device of the console.

The PCCT apparatus 30 is implemented with a remote reconstruction function that receives instructions relating to image reconstruction processing and the like from outside via the communication network 20 and executes processing according to the received instructions. The PCCT apparatus 30 receives an instruction to generate a material discrimination image from the AI server 14, and generates the material discrimination image according to the received instruction. A material discrimination image is an image in which a specific material is emphasized or suppressed. The term "material discrimination image" includes the concept of a material density image. The PCCT apparatus 30 can generate images that can discriminate between, for example, water, iodine, fat, calcium, and the like. The PCCT apparatus 30 is an example of a "CT apparatus" in the present disclosure.

The viewer server 16 is a server that provides information for display, including images for interpretation and AI results. By accessing the viewer server 16 from a client terminal such as the radiology department PC 18, an interpretation screen is displayed on the display device of the radiology department PC 18 or the like.

The radiology department PC 18 may be, for example, an image interpretation viewer terminal for the radiology department. Although only one radiology department PC 18 is shown in Fig. 1, a plurality of radiology department PCs 18 may be connected to the communication network 20.

The configuration of the information processing apparatus 10 shown in Fig. 1 is an example, and the specific forms of the PACS 12, the AI server 14, the viewer server 16, and the radiology department PC 18 are not limited. For example, the PACS 12, the AI server 14, the viewer server 16, and the radiology department PC 18 may be configured as an integrated unit, or a system may be constructed by combining some of the plurality of elements, or by further distributing processing functions.

The PACS 12, the AI server 14, and the viewer server 16 may be physical servers, virtual servers, or a combination of these. In addition, some or all of the PACS 12, the AI server 14, and the viewer server 16 may be implemented by cloud computing.

### Example of Hardware Configuration of Computer

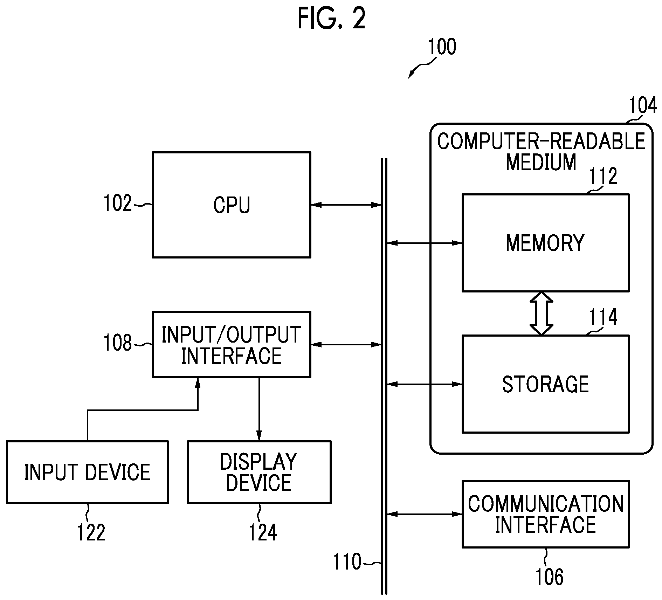

Fig. 2 is a block diagram showing an example of a hardware configuration of a computer 100 used in the information processing apparatus 10. Each of the PACS 12, the AI server 14, the viewer server 16, and the radiology department PC 18 shown in Fig. 1 may have a configuration similar to that of the computer 100, or some or all of the processing functions of the PACS 12, the AI server 14, the viewer server 16, and the radiology department PC 18 may be implemented in the computer 100.

The computer 100 includes a central processing unit (CPU) 102, a non-transitory tangible computer-readable medium 104, a communication interface 106, and an input/output interface 108. The computer 100 may include a graphics processing unit (GPU) (not shown).

The CPU 102 is connected to the computer-readable medium 104, the communication interface 106, and the input/output interface 108 via a bus 110. The computer-readable medium 104 includes a memory 112 which is a main storage device and a storage 114 which is an auxiliary storage device. The computer-readable medium 104 may be, for example, a semiconductor memory, a hard disk drive (HDD) device, or a solid state drive (SSD) device, or a combination of two or more of these. The computer-readable medium 104 stores various programs including an image processing program and a display control program, data, and the like.

The computer 100 may comprise an input device 122 and a display device 124. The input device 122 and the display device 124 are connected to the bus 110 via the input/output interface 108. The input device 122 is composed of, for example, a keyboard, a mouse, a multi-touch panel, another pointing device, a sound input device, or an appropriate combination thereof.

The display device 124 is composed of, for example, a liquid crystal display, an organic electro-luminescence (OEL) display, a projector, or an appropriate combination of these.

### Outline of Operations of Information Processing Apparatus 10 and PCCT Apparatus 30

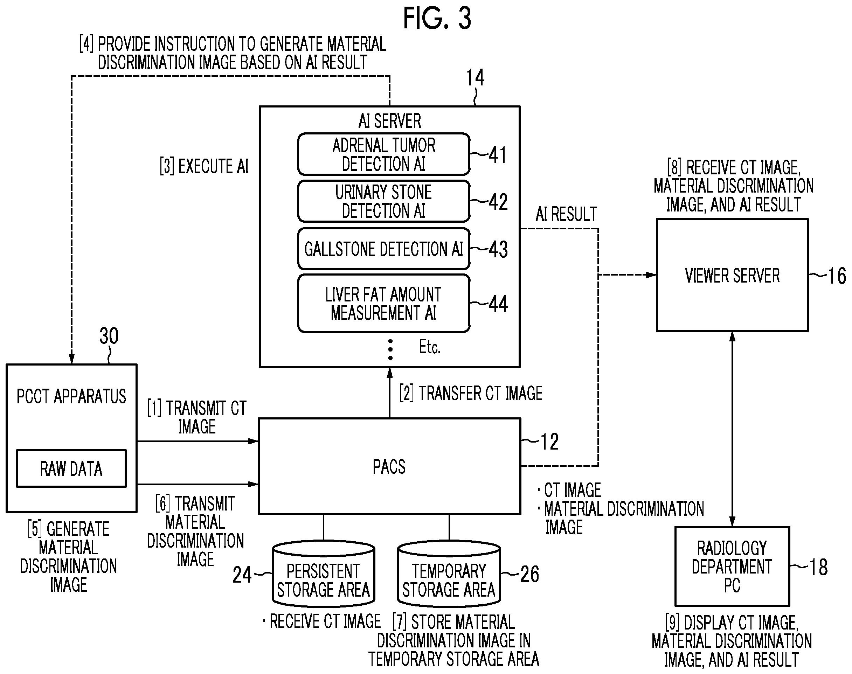

Fig. 3 shows an outline of the operations of the information processing apparatus 10 and the PCCT apparatus 30. The outline of the operations of this system will be described in the order of operations shown in [1] to [9] in Fig. 3.
[1] The PCCT apparatus 30 executes imaging in accordance with the order and transmits the obtained CT images to the PACS 12. The CT image is an example of a "first image" in the present disclosure.
[2] The PACS 12 transfers the CT images received from the PCCT apparatus 30 to the AI server 14. In addition, the PACS 12 stores the CT images received from the PCCT apparatus 30 in the persistent storage area 24.
[3] The AI server 14 executes a plurality of AI processes on the CT images acquired via the PACS 12. Specifically, the AI server 14 executes the analysis process including at least one process of detection of an adrenal tumor, detection of a urinary stone, detection of a gallstone, or calculation of a liver fat amount. The analysis process by the AI server 14 is performed for the purpose of detecting incidental findings.
[4] The AI server 14 instructs the PCCT apparatus 30 to generate a material discrimination image based on the AI results. The AI server 14 selects an image type that is more useful for diagnosis according to the AI results and instructs the PCCT apparatus 30 to generate the image type. Image types useful for diagnosis include concepts such as image types necessary for detailed image diagnosis, image types recommended for observation by the user, and image types expected to be observed by the user.
[5] The PCCT apparatus 30 receives a generation instruction from the AI server 14 and generates a material discrimination image in accordance with the generation instruction. The PCCT apparatus 30 performs a reconstruction process based on the raw data held in a storage device within the apparatus, and generates a material discrimination image. The reconstruction process corresponds to a generation process of generating a material discrimination image. The relationship between the AI results and the generated images may be, for example, as follows.
   <Example 1> In a case in which an adrenal tumor is detected by AI, the PCCT apparatus 30 generates a fat density image. The fat density image is an example of a "second image corresponding to a result of the analysis process" in the present disclosure.
   <Example 2> The PCCT apparatus 30 may change the slice thickness of the fat density image to be generated depending on the size of the adrenal tumor detected by AI. In a case in which the size of a detected adrenal tumor is small, it is preferable to set the slice thickness smaller (thinner) than in a case in which the size is large.
   <Example 3> The PCCT apparatus 30 may change the slice position and the number of slices of the fat density image to be generated depending on the position of the adrenal tumor detected by AI.
   For example, in a case in which an abdomen of a subject is imaged to obtain a total of 400 slices of tomographic images, and an adrenal tumor is detected by AI, it is possible to perform processing such as generating a fat density image of 20 slices near the detected adrenal tumor. In this way, by generating fat density images at slice positions limited to the adrenal tumor detected by AI, it is possible to selectively generate material discrimination images required for observation while suppressing the generation of unnecessary images. A material discrimination image represented by a fat density image is an example of a "second image" in the present disclosure.
[6] The PCCT apparatus 30 transmits the generated material discrimination image to the PACS 12.
[7] The PACS 12 stores the material discrimination image received from the PCCT apparatus 30 in the temporary storage area 26.
[8] The viewer server 16 receives the CT images and the material discrimination images from the PACS 12, and receives AI results for the CT images from the AI server 14. The viewer server 16 performs processing to display the received CT images, material discrimination images, and AI results on the interpretation screen of a display device. The CT images, material discrimination images, and AI results may all be displayed simultaneously, or only some of them may be selectively displayed. The content to be displayed may be changed in response to a display request from the radiology department PC 18.
[9] The radiology department PC 18 can access the viewer server 16, acquire display data from the viewer server 16, and display the CT images, the material discrimination images, and the AI results on a display device.

### Example of Flowchart

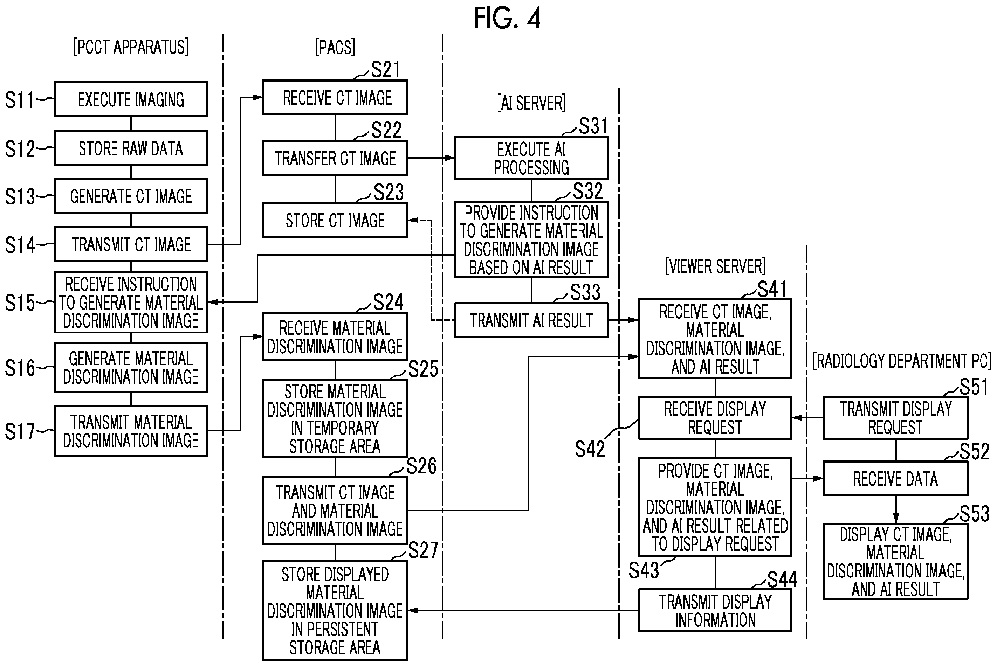

Fig. 4 is a flowchart showing an example of operations of the information processing apparatus 10 and the PCCT apparatus 30 according to the embodiment. The information processing method shown in the flowchart in Fig. 4 is an example of an "information processing method" in the present disclosure.

The PCCT apparatus 30 executes imaging of the subject in accordance with the order (Step S11), and stores the raw data obtained by imaging in a storage device (Step S12). Furthermore, the PCCT apparatus 30 reconstructs an image from the raw data by applying standard reconstruction conditions, and generates a CT image (Step S13). In Step S14, the PCCT apparatus 30 transmits the generated CT image to the PACS 12.

The PACS 12 receives the CT image from the PCCT apparatus 30 (Step S21), and transfers the received CT image to the AI server 14 (Step S22). In addition, the PACS 12 stores the received CT image in the persistent storage area 24 (Step S23). The order of Steps S22 and S23 may be changed.

In Step S31, the AI server 14 receives the CT image transferred from the PACS 12 and executes AI processing on the CT image. The AI server 14 applies some or all of the plurality of AI modules to analyze the CT image. The AI server 14 may select an AI module to be used for analyzing the CT image depending on the imaging part of the subject in the order. In addition to analysis related to the main complaint, using a plurality of AI modules to execute analysis such as detection or measurement from a plurality of viewpoints can be useful for detecting incidental findings.

In Step S32, the AI server 14 instructs the PCCT apparatus 30 to generate a material discrimination image based on the AI analysis results (AI results). Depending on the AI results, it may not be necessary to generate a material discrimination image. The AI server 14 determines whether or not a material discrimination image needs to be generated based on the AI results, and in a case in which generation is necessary, determines the image type to be generated and/or the reconstruction conditions, and transmits a generation instruction to the PCCT apparatus 30. In a case in which it is not necessary to generate a material discrimination image, the AI server 14 proceeds with the process of Step S33 without transmitting an instruction to generate a material discrimination image to the PCCT apparatus 30.

The PCCT apparatus 30 receives an instruction to generate a material discrimination image from the AI server 14 (Step S15). In a case in which the PCCT apparatus 30 receives an instruction to generate a material discrimination image from the AI server 14, the PCCT apparatus 30 generates a material discrimination image in accordance with the generation instruction (Step S16), and transmits the generated material discrimination image to the PACS 12 (Step S17).

The PACS 12 receives the material discrimination image generated by the PCCT apparatus 30 (Step S24), and stores the material discrimination image in the temporary storage area 26 (Step S25). Then, the PACS 12 transmits the CT image and the material discrimination image to the viewer server 16 (Step S26).

Further, the AI server 14 transmits the AI result to the viewer server 16 (Step S33). Further, the AI server 14 may also transmit the AI result to the PACS 12 in Step S33. The PACS 12 may store the AI result received from the AI server 14 in association with the CT images.

The viewer server 16 receives the CT image and the material discrimination image from the PACS 12, and receives the AI result from the AI server 14 (Step S41). In addition, the viewer server 16 may acquire the AI result via the PACS 12. The viewer server 16 can generate display data for an interpretation screen including at least one of the acquired CT image, material discrimination image, or AI result.

In Step S42, the viewer server 16 receives a display request from a client terminal such as the radiology department PC 18.

In Step S51, in a case in which a display request is transmitted from the radiology department PC 18 to the viewer server 16, the viewer server 16 receives this display request and provides display data for the interpretation screen including at least one of the CT image, the material discrimination image, or the AI result related to the display request to the radiology department PC 18 (Step S43).

The radiology department PC 18 receives data from the viewer server 16 (Step S52) and displays an interpretation screen including at least one of the CT image, the material discrimination image, or the AI result on the display device 124 (Step S53).

In addition, in Step S44, the viewer server 16 may transmit display information regarding images and the like actually displayed on the display device 124 of the radiology department PC 18 to the PACS 12. For example, in a case in which a display request for a material discrimination image is transmitted from the radiology department PC 18 to the viewer server 16 and the material discrimination image is displayed on the display device 124 of the radiology department PC 18, the viewer server 16 notifies the PACS 12 of display information indicating that the material discrimination image has been displayed (used for observation).

The PACS 12 receives the display information from the viewer server 16, moves the material discrimination image displayed on the radiology department PC 18 from the temporary storage area 26 to the persistent storage area 24, and stores the material discrimination image in the persistent storage area 24 in association with the CT image (Step S27). Among the material discrimination images stored in the temporary storage area 26, the material discrimination images that have not been used for display may be deleted from the temporary storage area 26 at an appropriate timing.

Furthermore, instead of transmitting display information from the viewer server 16 to the PACS 12, a command to move the target material discrimination image from the temporary storage area 26 to the persistent storage area 24 may be transmitted from the viewer server 16 to the PACS 12.

### About Processor

In the present embodiment, each process is executed by any computer. Furthermore, any computer may execute these processes using a processor, a program, or a combination thereof. Any computer may be a general purpose computer, a special purpose computer, a system such as a workstation, or other element of hardware capable of executing a program.

The processor may be configured by one or more pieces of hardware, and the type of hardware is not limited. For example, the processor may be configured with hardware such as a central processing unit (CPU), a micro processing unit (MPU), a programmable logic device such as a field-programmable gate array (FPGA), a dedicated circuit for executing specific processing such as an application-specific integrated circuit (ASIC), a graphic processing unit (GPU), or a neural processing unit (NPU).

The processor also has various units or means for executing various processes in the present embodiment. Additionally, the type of hardware may be a combination of different types of hardware. In a case in which a plurality of pieces of hardware are configured to execute one or more processes of a certain processor, the plurality of pieces of hardware may be present in devices physically separate from each other, or may be present in the same device. In addition, in any of the embodiments, the order of the processes performed by the processor is not limited to the order described above, and may be changed as appropriate. The hardware is composed of electrical circuits (circuitry) combining circuit elements such as semiconductor elements.

Furthermore, the present embodiment may be implemented by hardware, software, firmware, microcode, or a combination thereof. The software, firmware, and microcode are constituted by programs. Also, the program may be, for example, a group of program modules, each function of which may be implemented by a processor configured to execute each function. The program may be a program code or a number of code segments stored in one or more non-transitory computer-readable media (for example, a storage medium or other storage). The program may be stored in a plurality of non-transitory computer-readable media that are present in devices physically separate from each other in a divided manner. A Program code or a code segment may represent a procedure, a function, a subprogram, a routine, a subroutine, a module, a software package, a class, or any combination of instructions, data structures, or program statements. A program code or a code segment may be connected to another code segment or a hardware circuit by transmitting or receiving information, data, arguments, parameters, or memory contents.

### About Program that operates Computer

It is possible to record a program causing a computer to implement some or all of the processing functions of the information processing apparatus 10, on an optical disc, a magnetic disk, or a computer-readable medium such as a semiconductor memory or other tangible non-transitory information storage medium, and to provide the program through this information storage medium.

In addition, instead of the aspect in which the program is stored and provided in such a tangible non-transitory information storage medium, a program signal can be provided as a download service by using an electric telecommunication line, such as the Internet.

Further, some or all of the processing functions in the information processing apparatus 10 may be implemented by cloud computing or may be provided as software as a service (SaaS).

### Advantages of Embodiment

The present embodiment has the following advantages.
[1] According to the information processing apparatus 10, since unnecessary material discrimination images are not generated, the image capacity of the PACS 12 can be reduced.
[2] According to the information processing apparatus 10, since unnecessary material discrimination images are not generated, traffic on the communication network 20 can be reduced.
[3] According to the information processing apparatus 10, since the material discrimination image to be generated is automatically determined using AI, the work of the service staff, such as setting the generation condition of the material discrimination image, can be reduced.
[4] According to the information processing apparatus 10, a material discrimination image useful for diagnosis is automatically selected based on the AI results, and an instruction to generate the image is automatically issued to the PCCT apparatus 30. Therefore, the time required to check the image can be shortened compared to the case in which the material discrimination image that the user wishes to observe is generated on demand. Accordingly, it is possible to perform interpretation with high time efficiency.

### Modification Example 1

The learning device provided in the AI server 14 is not limited to the learning device shown in Figs. 1 and 3, but may include a trained model that causes a processor to execute a process of detecting lesions from an input image. The learning device provided in the AI server 14 may include a trained model that causes the processor to execute a process of detecting at least one of a tumor, a nodule, a stone, fat, or a calcified area from an input image.

### Modification Example 2

In the above embodiment, an example has been described in which an instruction to generate a material discrimination image is provided to the PCCT apparatus 30, but the present invention is not limited to the PCCT apparatus 30, and may be configured to instruct a general CT apparatus equipped with a detector that detects X-ray transmission data in integral units to generate a CT image by an image reconstruction process under different reconstruction conditions.

For example, a CT apparatus replacing the PCCT apparatus 30 performs an image reconstruction process by applying a first reconstruction condition to raw data obtained by imaging, thereby generating a first reconstructed image. This first reconstructed image is transmitted to the PACS 12 and transferred to the AI server 14. Based on the analysis results (AI results) of the first reconstructed image, the AI server 14 instructs the CT apparatus to generate a second reconstructed image by applying a second reconstruction condition different from the first reconstruction condition. Upon receiving this generation instruction, the CT apparatus performs an image reconstruction process by applying the second reconstruction condition to the raw data in the apparatus, and generates a second reconstructed image.

The second reconstructed image may be, for example, an image having a thinner slice thickness (higher resolution) than the first reconstructed image. That is, the first reconstructed image may be a CT image (simple CT image) having a relatively thick slice thickness, which is referred to as a so-called "thick slice", and the second reconstructed image may be a CT image having a relatively thin slice thickness, which is referred to as a so-called "thin slice". The "CT image" and the "material discrimination image" in the embodiment described with reference to Figs. 1 to 4 can be understood by replacing them with the first reconstructed image and the second reconstructed image. Furthermore, two or more types of images of a plurality of image types may be generated as the second reconstructed image.

### Others

The present disclosure is not limited to the above-described embodiment, and various modifications are possible without departing from the spirit of the technical idea of the present disclosure.

### Explanation of References

10: information processing apparatus
12: PACS
14: AI server
16: viewer server
18: radiology department PC
20: communication network
24: persistent storage area
26: temporary storage area
30: PCCT apparatus
41: adrenal tumor detection AI
42: urinary stone detection AI
43: gallstone detection AI
44: liver fat amount measurement AI
100: computer
102: CPU
104: computer-readable medium
106: communication interface
108: input/output interface
110: bus
112: memory
114: storage
122: input device
124: display device
S11 to S17: steps of operation of PCCT apparatus
S21 to S27: steps of operation of PACS
S31 to S33: steps of operation of AI server
S41 to S44: steps of operation of viewer server
S51 to S53: steps of operation of radiology department PC

## Claims

1. An information processing apparatus (10) comprising a processor (102),
wherein the processor (102) is configured to:
acquire a first image captured by a CT apparatus (30);
execute an analysis process of the first image using a learning device;
cause the CT apparatus (30) to execute a generation process of a second image corresponding to a result of the analysis process; and
display at least one of the first image, the result of the analysis process, or the second image on a display device (124).

2. The information processing apparatus (10) according to claim 1,
wherein the first image is a CT image, and
the second image is a material discrimination image.

3. The information processing apparatus (10) according to claim 1,
wherein the second image is an image generated by an image reconstruction process under a reconstruction condition different from a reconstruction condition of the first image.

4. The information processing apparatus (10) according to any one of claims 1 to 3,
wherein the processor (102) is configured to output an instruction to the CT apparatus (30) to generate the second image based on the result of the analysis process.

5. The information processing apparatus (10) according to any one of claims 1 to 4,
wherein the processor (102) is configured to set a generation condition of the second image according to the result of the analysis process.

6. The information processing apparatus (10) according to any one of claims 1 to 5,
wherein the learning device includes a trained model that causes the processor (102) to execute a process of detecting a lesion from an input image.

7. The information processing apparatus (10) according to any one of claims 1 to 6,
wherein the learning device includes a trained model that causes the processor (102) to execute a process of detecting at least one of a tumor, a nodule, a stone, fat, or a calcified area from an input image.

8. The information processing apparatus (10) according to any one of claims 1 to 7,
wherein the analysis process includes at least one process of detection of an adrenal tumor, detection of a urinary stone, detection of a gallstone, or calculation of a liver fat amount.

9. The information processing apparatus (10) according to any one of claims 1 to 8,
wherein the processor (102) is configured to, in a case in which an adrenal tumor is detected by the analysis process, cause the CT apparatus (30) to execute a generation process of a fat density image as the second image.

10. The information processing apparatus (10) according to claim 9,
wherein the processor (102) is configured to set a slice thickness of the fat density image generated by the generation process according to a size of the adrenal tumor detected by the analysis process,
and/or,
wherein the processor (102) is configured to set a slice position and the number of slices of the fat density image generated by the generation process according to a position of the adrenal tumor detected by the analysis process.

11. The information processing apparatus (10) according to any one of claims 1 to 10,
wherein the processor (102) is configured to:
receive a display request for the second image via an input device (122); and
display the second image on the display device (124) based on the display request.

12. The information processing apparatus (10) according to any one of claims 1 to 11, further comprising a storage device including a temporary storage area (26) and a persistent storage area (24),
wherein the processor (102) is configured to:
store the first image in the persistent storage area (24); and
store the second image in the temporary storage area (26).

13. The information processing apparatus (10) according to claim 12,
wherein the processor (102) is configured to store the second image displayed on the display device (124) in the persistent storage area (24),
and/or,
wherein the processor (102) is configured to store, in the persistent storage area (24), only an image displayed on the display device (124) among the second images stored in the temporary storage area.

14. An information processing method executed by a processor (102), the information processing method comprising:
a step of acquiring a first image captured by a CT apparatus (30);
a step of executing an analysis process of the first image using a learning device;
a step of causing the CT apparatus (30) to execute a generation process of a second image corresponding to a result of the analysis process; and
a step of displaying at least one of the first image, the result of the analysis process, or the second image on a display device (124).

15. A non-transitory, computer-readable recording medium which records thereon, a program that causes a processor (102) to implement the information processing method according to claim 14.
